# EUROPÄISCHE PATENTSCHRIFT

(11) **EP 1 153 002 B1**
(45) Veröffentlichungstag und Bekanntmachung des Hinweises auf die Patenterteilung: **17.09.2014**
(21) Anmeldenummer: 00905025.3
(22) Anmeldetag: 04.02.2000
(51) Int. Cl.: C04B 41/82, C04B 41/88, A61K 6/06

(54) **EINFÄRBUNG VON DENTALKERAMIKEN MITTELS IONISCHER ODER KOMPLEXHALTIGER LÖSUNGEN**
COLORING DENTAL CERAMICS BY WAY OF IONIC OR COMPLEX-CONTAINING SOLUTIONS
COLORATION DE CERAMIQUES DENTAIRES AU MOYEN DE SOLUTIONS IONIQUES OU RENFERMANT DES COMPLEXES

(30) Priorität: 04.02.1999 DE 19904522
(43) Veröffentlichungstag der Anmeldung: 14.11.2001
(60) Teilanmeldung: 10168296.1
(73) Patentinhaber: 3M Deutschland GmbH, 41453 Neuss (DE)
(72) Erfinder: SUTTOR, Daniel, D-82229 Seefeld (DE); HAUPTMANN, Holger, D-82404 Sindelsdorf (DE); SCHNAGL, Robert, D-86899 Landsberg (DE); FRANK, Sybille, D-82229 Seefeld (DE)
(74) Vertreter: Vossius & Partner
(86) Internationale Anmeldenummer: PCT/EP2000/000910
(87) Internationale Veröffentlichungsnummer: WO 2000/046168

(56) Entgegenhaltungen:
- EP-A- 0 329 565
- DE-A- 3 424 777

## Beschreibung

Die Erfindung betrifft die Einfärbung von Dentalkeramiken mittels ionischer oder komplexhaltiger Lösungen. Im besonderen betrifft die Erfindung die Einfärbung von Dentalkeramiken auf Zirkonoxidbasis mittels Lösungen von Seltenerdmetallen und Nebengruppenelementen.

Keramiken werden aufgrund ihrer physikalischen Eigenschaften bei der Erstellung von hochwertigem Zahnersatz sehr geschätzt. Aluminium- und Zirkonoxidkeramiken sind im Medizinbereich seit langem die Materialien der Wahl (Sonderdruck aus Industrie Diamanten Rundschau, IDR 2/1993, "Aluminium- und Zirkonoxidkeramik in der Medizin"). Im Dentalbereich gibt es eine Vielzahl von Publikationen, die sich mit der Verwendung von Keramiken zur Herstellung von Prothesen beschäftigen. Ebenso sind diverse Keramiksysteme bereits auf dem Dentalmarkt verfügbar (CEREC, Fa. Siemens; Procera, Fa. Nobel-Biocare).

Die EP 0 329 565 A1 beschreibt eine Halterung (Klammer) aus Sinterkeramik auf Basis von durch Zusatz an einer geringen Menge eines Oxids teilweise stabilisiertem Zirkoniumoxid, wobei die Klammer nicht porös ist und ihr nach dem Sintervorgang ein deutlich den Zähnen entsprechendes Aussehen und eine entsprechende Durchsichtigkeit aufgrund des ergänzenden Zusatzes an Übergangsmetalloxiden verliehen wird.

Insbesondere im Dentalbereich spielen aber nicht nur die mechanischen Parameter eine große Rolle, sondern besonders auch die Ästhetik. Transluzenz und Farbgebung der Gerüst- oder Verblendkeramiken sind wichtig, um dem Patienten ein natürliches Aussehen seines künstlichen Zahnersatzes zu ermöglichen.

Zahnersatz wird üblicherweise aus einem Gerüst und einer Verblendung hergestellt.

Bei den bisher bekannten Systemen kann nur eine oberflächliche individuelle Einfärbung des Grundgerüstes durch den Zahntechniker vorgenommen werden, dabei sind die ästhetischen Gestaltungsmöglichkeiten eingeschränkt.

Um ein natürliches Erscheinungsbild der Prothese zu erzielen, muß die Zahnfarbe und die Transluzenz über mehrere Schichten hinweg, beginnend mit dem Gerüst, simuliert werden.

Das natürliche Erscheinungsbild einer Prothese wird gewährleistet durch eine möglichst hohe freie Weglänge z = x + y + m des einfallenden Lichtes durch die Schicht (x) der Verblendkeramik und Schicht (m) der Gerüstkeramik und ggf. einer Zwischenschicht (y).

Herkömmliche Systeme müssen zur Veränderung des Grundfarbtones der Gerüstkeramik mit färbenden Zwischenschichten, beispielsweise Opaquer-Linern, arbeiten, die keine oder stark verringerte Transluzenz aufweisen; die freie Weglänge des Lichtes verringert sich um die Dicke der Gerüstkeramik (m) und der Zwischenschicht (y) auf z = x. Eine Beschreibung dieser Vorgehensweise ist z.B. in den Gebrauchsinformationen der Fa. Vita zum System Vita-Dur α oder der Fa. DUCERA mit dem System ALL Ceram zu finden.

Derartige Systeme verwenden als Zwischenschicht Farbpasten bzw. Farbsuspensionen, die in mehreren Arbeitsgängen vom Zahntechniker auf das Gerüst aufgebracht und abschließend im Ofen gebrannt werden.

Dieser Vorgang ist nicht nur zeitaufwendig, sondern auch kostenintensiv.

Der Erfindung liegt somit die Aufgabe zugrunde, ein System zur Einfärbung von Dentalkeramik, insbesondere keramischem Zahnersatz, bereitzustellen, das eine optimale Ästhetik bei minimalem Arbeitsaufwand und bei minimierten Kosten gewährleistet.

Überraschend wird diese Aufgabe gelöst durch ein Verfahren zum Einfärben von Dentalkeramiken im porösen oder saugfähigen Zustand, das dadurch gekennzeichnet ist, dass die Keramiken transluzent sind und zum Einfärben Metallionen-Lösungen oder Metallkomplex-Lösungen verwendet werden. Hierfür bevorzugte Lösungen enthalten definierte Konzentrationen mindestens eines der Salze oder Komplexe der Seltenerden-Elemente oder der Elemente der Nebengruppen.

Die Lösungen sind vorzugsweise auf wäßriger oder alkoholischer Basis. Geeignete Salze oder Komplexe sind bevorzugt solche aus der Gruppe der Seltenerden oder der II. oder VIII. Nebengruppe, insbesondere Pr, Er, Fe, Co, Ni, Cu.

Bevorzugt sind Salze oder Komplexe mit anorganischen Gegenionen wie z. B. Cl⁻, Br, J⁻, SO₄ ²⁻, SO₃²⁻, NO₂⁻, NO₃⁻, ClO₄⁻, ONC⁻, SCN⁻ wobei auch Oxokomplexe saurer oder basischer Salze gemeint sein können, nicht aber Doppelsalze mit einem Element der 1. oder 2. Hauptgruppe. Desweiteren sind Salze oder Komplexe mit organischen Ionen oder Liganden bevorzugt, die 1 bis 30 C-Atome und eine Anzahl von 1 bis 10 Heteroatome, wie O, N, S, enthalten. Im einzelnen sind dies Alkoxide oder Salze organischer Säuren. Bevorzugt sind hier unter den Alkoxiden die Salze der C₁-C₁₀-Alkanole, insbesondere die Methoxide, Ethoxide, n- und i-Propoxide und n-, i-, sek- bzw. tert-Butoxide. Unter den Salzen organischer Säuren sind diejenigen von Mono-, Di- und Tri- C₁-C₂₀-Carbonsäuren bevorzugt, insbesondere Formiat, Acetat, Malat, Maleat, Maleinat, Tartrat, Oxalat. Zuletzt sind auch Komplexbildner unter den Liganden zu verstehen, die dazu dienen, die Metallsalze in ihrer Oxidationsstufe und in Lösung zu stabilisieren. Diese können organische C₂-C₂₀-Moleküle mit bis zu 10 Heteroatomen O, N oder S, darunter insbesondere EDTA und seine Salze, NTA, Salicylsäure, Phenole, 5-Sulfosalicylsäure etc., sein.

Bevorzugt sind wäßrige oder alkoholische Lösungen von Pr, Er, Fe, beispielsweise als Chloride, Acetate oder Alkoholate.

Die Ionen oder Komplexe werden vorzugsweise in Konzentrationen von 0,0001 bis 15 Gew.-%, besonders bevorzugt von 0,001 bis 10 Gew.-% und ganz besonders bevorzugt von 0,01 bis 7 Gew.-% eingesetzt.

Unter Dentalkeramiken werden hier alle hochfesten Oxide der Elemente der Hauptgruppen II, III und IV und der Nebengruppen III und IV sowie deren Mischungen verstanden, insbesondere Al₂O₃, ZrO₂, sowohl, teil- als auch vollstabilisiert, MgO, TiO₂ und deren Mischungen. Insbesondere werden unter Keramiken und Dentalkeramiken transluzente Keramiken verstanden.

Überraschend hierbei ist ferner, daß die Farbtiefe der Einfärbung nicht von der Einwirkdauer der Lösung, sondern nur von deren Konzentration abhängig ist. Dies ist besonders vorteilhaft, da der Zahntechniker nicht auf sekundengenaue Einwirkzeiten fixiert ist, sondern seine Arbeiten innerhalb gewisser Toleranzen beliebig lange mit den erfindungsgemäßen Lösungen behandeln kann. Die Einwirkdauer der Lösung kann theoretisch beliebig lange sein. Sie ist nur abhängig von anderen Effekten in der Lösung, beispielsweise pH-Wert-Änderungen oder Freisetzung von Ionen, die den Färbevorgang behindern können. So ergibt sich im allgemeinen eine Einwirkdauer, bis zu der die Farbtiefe der Einfärbung sich nicht verändert, von einigen Stunden. Die Einwirkdauer beträgt vorzugsweise unter 2 Stunden, insbesondere unter 1 Stunde und besonders bevorzugt unter 20 Minuten.

Vorteilhafterweise kann durch die vorliegende Erfindung die oben erwähnte Zwischenschicht (y) komplett entfallen, da bereits die Gerüstkeramik durch die erfindungsgemäßen Lösungen individuell eingefärbt werden kann. Es entfällt daher ein zusätzlicher kosten- und zeitintensiver Schritt des Aufbrennens der Zwischenschicht. Dem einfallenden Licht steht nun die freie Weglänge z = x + y + m zur Verfügung, da der Weg nicht mehr durch die Zwischenschicht unterbrochen wird.

Die erfindungsgemäßen Lösungen können neben den Salzen oder Komplexen der Seltenerden-Elemente oder der Nebengruppenelemente auch Stabilisierungsmittel, wie Komplexbildner, Malhilfsmittel sowie organische Farbpigmente zur Erleichterung der Farbabstimmung durch den Zahntechniker enthalten.

Als Stabilisierungsmittel geeignet sind Komplexbildner, wie Ethylendiamintetraessigsäure. Unter Malhilfsmitteln sind beispielsweise temporäre Bindemittel und Thixotropiemittel, wie Polyglykole, Polysaccharide, Polyethylenglykole, Polyvinylalkohole, hydrierte Rizinusöle, zu verstehen.

Aufgrund der niedrigen Konzentrationen an farbgebenden Ionen oder Komplexen innerhalb der erfindungsgemäßen Lösungen und der damit verbundenen schlechten optischen Erkennbarkeit des aufgebrachten Farbtons, können auch organische Pigmente zur Erleichterung der Farbabstimmung durch den Zahntechniker zugesetzt werden. Besonders hilfreich sind diese Zusätze bei der bereichsweisen Applikation der Lösungen über Applikationsinstrumente. Die Zusätze sind so zu wählen, daß sie beim Brennen der prothetischen Arbeit rückstandsfrei zerstört werden.

Die erfindungsgemäßen Lösungen können auf folgende Weisen auf die vorgesinterten bzw. saugfähigen Keramiken aufgebracht werden:
1. Tauchen der Dentalkeramik in Lösungen definierter Konzentrationen;
2. Auftragen der Lösungen auf die Keramik mittels geeigneter Applikationsinstrumente, beispielsweise Pinsel, Tupfer;
3. Auftragen der Lösungen auf die Keramik mittels Sprühverfahren.

Mit dem erfindungsgemäßen Verfahren können Wandstärken von bis zu 10 mm, bevorzugt 7 mm, durchgehend eingefärbt werden. Insbesondere im dentalen Bereich bei der Herstellung von Kronen und Brücken sind Abmessungen von 10 mm, bevorzugt 7 mm, für den Durchmesser eines Werkstückes und 7 mm, bevorzugt 5 mm, für die Höhe eines Werkstückes möglich. Diese mm-Angaben beziehen sich auf die Dicken der einfärbbaren Wandstärken der dentalen Werkstücke. Natürlich sind auch Werkstücke außerhalb der hier angegebenen Grenzen im Umfang der Erfindung enthalten.

Vorzugsweise werden die Dentalkeramiken vollständig durchgefärbt.

Die Erfindung wird nachfolgend durch Beispiele näher erläutert, ohne daß sie dadurch beschränkt sein soll.

### Konzentrationsabhängige Einfärbung von durch 3 Mol Yttriumoxid stabilisiertem Zirkonoxid

Zur Herstellung der Lösungen werden die entsprechenden Mengen Farbreagenz in Wasser gelöst. Keramikkörper werden darin 5 min tauchgebadet und anschließend getrocknet und gesintert. Die Proben werden anschließend für die Farbmessung geschliffen und poliert. Der Farbbestimmung liegen folgende Parameter zugrunde:

| | |
|---|---|
| Opazitätswert O: | Maß für die Transparenz (0% ist voll transparent, 100% ist opak); |
| L*-Wert: | Helligkeit (100: vollständige Reflexion; 0: keine Reflexion); |
| a*-Wert: | Rot-Grünverschiebung (+a: rot; -a: grün); |
| b*-Wert: | Gelb-Blauverschiebung (+b: gelb; -b: blau); |

| | |
|---|---|
| Meßgerät: Fa. HunterLab, LabScan Spectrocolorimeter; Meßmethode: Cielab (Farbe); Opazität nach ASTM D2805 / TAPPI T425 / TAPPI T519. | |

Zum Nachweis der Unabhängigkeit der Farbintensität von der Einwirkdauer der Lösung werden bei fester Lösungskonzentration verschiedene Einwirkzeiten zugrundegelegt und die Farbbestimmung analog durchgeführt.

Als Material wurde käufliche Zirkonoxidkeramik der Firma Tosoh, Japan vom Typ TZ3YE verwendet.

### Einfärbung mit Fe(III)Cl₃-Lösungen

| **Konzentration Lösung [Gew.-%]** | **L*** | **a*** | **b*** | **O** |
|---|---|---|---|---|
| | | | | |
| **0** | 85,67 | -0,97 | 1,51 | 91,4 |
| **0,1** | 83,93 | -1,67 | 5,15 | 92,36 |
| **0,3** | 79,04 | -1,52 | 22,35 | 95,1 |
| **0,5** | 75,37 | 1,16 | 25 | 95,32 |
| **0,75** | 74,01 | 1,72 | 25,91 | 96,51 |
| **1** | 72,25 | 2,83 | 24,67 | 97,79 |

### Einfärbung mit Pr(III)Acetat-Lösungen

| **Konzentration Lösung [Gew.-%]** | **L*** | **A*** | **b*** | **O** |
|---|---|---|---|---|
| | | | | |
| **0,1** | 81,02 | -3,60 | 224,98 | 89,98 |
| **0,25** | 80,80 | -3,02 | 34,17 | 91,40 |
| **0,75** | 74,85 | 4,77 | 47,31 | 92,11 |

**Ergebnis:** Über die Konzentration der Lösung kann die Intensität der Farbe gesteuert werden.

### abhängigkeit der Farbintensität von der Einwirkdauer

### Lösungskonzentration: 0,75 Gew.-% Fe (III) Cl - Lösung

| **Einwirkdauer** | **L*** | **a*** | **b*** | **O** |
|---|---|---|---|---|
| | | | | |
| **2 Minuten** | 75,18 | 0.32 | 20,15 | 96,05 |
| **5 Minuten** | 76,06 | -0,42 | 21,4 | 95,86 |
| **10 Minuten** | 75,18 | -0,09 | 22,4 | 96,08 |
| **20 Minuten** | 75,80 | -0,21 | 23,11 | 96,37 |

**Ergebnis:** Die Einwirkdauer hat keinen Einfluß auf die Farbintensität.

## Patentansprüche

1. Verfahren zum Einfärben von Dentalkeramiken im porösen oder saugfähigen Zustand, **dadurch gekennzeichnet, dass** die Keramiken transluzent sind und zum Einfärben Metallionen-Lösungen oder Metallkomplex-Lösungen verwendet werden.

2. Verfahren nach Anspruch 1, **dadurch gekennzeichnet, dass** die Lösungen mindestens eines der Ionen oder Komplexe der Seltenerden-Elemente oder Nebengruppen enthalten.

3. Verfahren nach Anspruch 2, **dadurch gekennzeichnet, dass** die Lösungen Pr, Er, Fe, Co, Ni oder Cu enthalten.

4. Verfahren nach Anspruch 2 oder 3, **dadurch gekennzeichnet, dass** als Salze Chloride, Acetate oder Alkohole sowie Oxokomplexe verwendet werden.

5. Verfahren nach Anspruch 4, wobei als Gegenionen Cl⁻, Br⁻, J⁻, SO₄²⁻, SO₃²⁻, NO₂⁻, NO₃⁻, ClO₄⁻, ONC⁻, SCN⁻, Formiat, Acetat, Malat, Maleat, Maleinat, Tartrat, Oxalat, Methoxid, Ethoxid, n-Propoxide, i-Propoxid, n-Butoxid, i-Butoxid, sek-Butoxid oder tert-Butoxid verwendet werden.

6. Verfahren nach mindestens einem der Ansprüche 1 bis 5, **dadurch gekennzeichnet, dass** Dentalkeramiken im vorgesinterten Zustand verwendet werden.

7. Verfahren nach mindestens einem der Ansprüche 1 bis 6, **dadurch gekennzeichnet, dass** Dentalkeramiken auf Zirkonoxid- oder Aluminiumoxidbasis verwendet werden.

8. Verfahren nach mindestens einem der Ansprüche 1 bis 7, **dadurch gekennzeichnet, dass** die ionischen oder komplexhaltigen Lösungen auf wässriger oder alkoholischer Basis sind.

9. Verfahren nach einem' der Ansprüche 1 bis 8, wobei die Lösungen Stabilisierungsmittel, Malhilfsmittel oder organische Farbpigmente enthalten.

10. Verfahren nach mindestens einem der Ansprüche 1 bis 9, **dadurch gekennzeichnet, dass** die Einwirkdauer der ionischen oder komplexhaltigen Lösungen im Stundenbereich, insbesondere unter 2 Stunden, ganz besonders unter 1 Stunde und besonders bevorzugt unter 20 Minuten liegt.

11. Verfahren nach mindestens einem der Ansprüche 1 bis 10, **dadurch gekennzeichnet, dass** die Konzentration der Lösungen 0,001 bis 15 Gew.-% beträgt.

12. Verfahren nach mindestens einem der Ansprüche 1 bis 11, **dadurch gekennzeichnet, dass** das Einfärben durch Tauchen der Keramik in die Lösungen, durch Auftragen der Lösungen auf die Keramik mit Hilfe von Applikationsinstrumenten oder durch Aufsprühen der Lösungen auf die Keramik erfolgt.

13. Verfahren nach mindestens einem der Ansprüche 1 bis 12, **dadurch gekennzeichnet, dass** die einzufärbenden Keramiken einen Durchmesser von 10 mm, vorzugsweise 7 mm, und eine Höhe von 7 mm, vorzugsweise 5 mm, besitzen.

14. Verfahren nach mindestens einem der Ansprüche 1 bis 13, **dadurch gekennzeichnet, dass** die Keramiken vollständig durchgefärbt werden.

15. Verfahren nach einem der Ansprüche 1 bis 14, wobei das Verfahren bei der Herstellung von Kronen oder Brücken verwendet wird.

## Claims

1. A process for coloring dental ceramics in the porous or absorbent state, **characterized in that** the ceramics are translucent and metal ion solutions or metal complex solutions are used for the coloring.

2. The process according to Claim 1, **characterized in that** the solutions contain at least one of the ions or complexes of the rare earth elements or subgroups.

3. The process according to Claim 2, **characterized in that** the solutions contain Pr, Er, Fe, Co, Ni or Cu.

4. The process according to Claim 2 or 3, **characterized in that** chlorides, acetates or alcohols, as well as oxo complexes, are used as salts.

5. The process according to Claim 4, wherein Cl⁻, Br⁻, I-, SO₄²⁻, SO₃²⁻, NO₂⁻, NO₃⁻, ClO₄⁻, ONC⁻, SCN⁻, formate, acetate, malate, maleate, maleinate, tartrate, oxalate, methoxide, ethoxide, n-propoxides, i-propoxide, n-butoxide, i-butoxide, sec-butoxide, or tert-butoxide are used as counterions.

6. The process according to at least one of Claims 1 through 5, **characterized in that** dental ceramics in the pre-sintered state are used.

7. The process according to at least one of Claims 1 through 6, **characterized in that** dental ceramics based on zirconium oxide or on aluminum oxide are used.

8. The process according to at least one of Claims 1 through 7, **characterized in that** the ionic solutions or solutions containing complex are water-based or alcohol-based.

9. The process according to any one of Claims 1 through 8, wherein the solutions contain stabilizing agents, coloring aids or organic color pigments.

10. The process according to at least one of Claims 1 through 9, **characterized in that** the duration of action of the ionic solutions or solutions containing complex is in the range of hours, in particular less than 2 hours, quite particularly less than 1 hour, and particularly preferably less than 20 minutes.

11. The process according to at least one of Claims 1 through 10, **characterized in that** the concentration of the solutions is 0.001 to 15 wt. %.

12. The process according to at least one of Claims 1 through 11, **characterized in that** the coloring takes place via immersion of the ceramic in the solutions, via application of the solutions onto the ceramic with the help of application instruments, or via spraying of the solutions onto the ceramic.

13. The process according to at least one of Claims 1 through 12, **characterized in that** the ceramics to be colored have a diameter of 10 mm (advantageously 7 mm) and a height of 7 mm (advantageously 5 mm).

14. The process according to at least one of Claims 1 through 13, **characterized in that** the ceramics are completely colored through.

15. The process according to at least one of Claims 1 through 14, wherein the process is used in the production of crowns or bridges.

## Revendications

1. Procédé pour la coloration de céramiques dentaires à l'état poreux ou absorbant, **caractérisé en ce que** les céramiques sont translucides et qu'on utilise, pour la coloration, des solutions d'ions métalliques ou de solutions de complexes métalliques.

2. Procédé selon la revendication 1, **caractérisé en ce que** les solutions contiennent au moins un des ions ou des complexes des éléments des terres rares ou des groupes secondaires.

3. Procédé selon la revendication 2, **caractérisé en ce que** les solutions contiennent Pr, Er, Fe, Co, Ni ou Cu.

4. Procédé selon la revendication 2 ou 3, **caractérisé en ce qu'**on utilise, comme sels, des chlorures, des acétates ou des alcools ainsi que des complexes oxo.

5. Procédé selon la revendication 4, dans lequel on utilise, comme contre-ions, Cl-, Br⁻, J⁻, SO₄²⁻, SO₃²⁻, NO₂⁻, NO₃⁻, CIO₄⁻, ONC⁻, SCN⁻, formiate, acétate, malate, maléate, maléinate, tartrate, oxalate, méthoxyde, éthoxyde, n-propoxyde, i-propoxyde, n-butoxyde, i-butoxyde, sec-butoxyde ou tert-butoxyde.

6. Procédé selon au moins l'une des revendications 1 à 5, **caractérisé en ce qu'**on utilise des céramiques dentaires à l'état préfritté.

7. Procédé selon au moins l'une des revendications 1 à 6, **caractérisé en ce qu'**on utilise des céramiques dentaires à base d'oxyde de zirconium ou d'oxyde d'aluminium.

8. Procédé selon au moins l'une des revendications 1 à 7, **caractérisé en ce que** les solutions ioniques ou contenant des complexes sont à base d'eau ou d'alcool.

9. Procédé selon l'une des revendications 1 à 8, dans lequel les solutions contiennent des stabilisants, des adjuvants de coloration ou des pigments colorés organiques.

10. Procédé selon au moins l'une des revendications 1 à 9, **caractérisé en ce que** la durée d'action des solutions ioniques ou contenant des complexes se situe dans la plage des heures et est en particulier inférieure à 2 heures, tout particulièrement inférieure à 1 heure et de manière particulièrement préférée inférieure à 20 minutes.

11. Procédé selon au moins l'une des revendications 1 à 10, **caractérisé en ce que** la concentration des solutions est de 0,001 à 15 % en poids.

12. Procédé selon au moins l'une des revendications 1 à 11, **caractérisé en ce que** la coloration est réalisée par immersion de la céramique dans les solutions, par application des solutions sur la céramique à l'aide d'instruments d'application ou par pulvérisation des solutions sur la céramique.

13. Procédé selon au moins l'une des revendications 1 à 12, **caractérisé en ce que** les céramiques à colorer présentent un diamètre de 10 mm, de préférence de 7 mm et une hauteur de 7 mm, de préférence de 5 mm.

14. Procédé selon au moins l'une des revendications 1 à 13, **caractérisé en ce que** les céramiques sont totalement colorées.

15. Procédé selon l'une des revendications 1 à 14, dans lequel le procédé est utilisé pour la fabrication de couronnes ou de bridges.
